# EUROPÄISCHE PATENTSCHRIFT

(11) **EP 3 592 836 B1**
(45) Veröffentlichungstag und Bekanntmachung des Hinweises auf die Patenterteilung: **06.04.2022**
(21) Anmeldenummer: 18729324.6
(22) Anmeldetag: 06.03.2018
(51) Int. Cl.: C12M 1/00, C12M 1/34, C12P 3/00, C12P 7/04

(54) **VERFAHREN ZUR BIOREAKTIVEN EXTRAKTION ERZEUGTEN SAUERSTOFFS AUS EINEM REAKTIONSRAUM, SOWIE VERWENDUNG VON PHOTOTROPHEN MIKROORGANISMEN BEI DER GEWINNUNG VON WASSERSTOFF**
PROCESS FOR THE BIOREACTIVE EXTRACTION OF PRODUCED OXYGEN FROM A REACTION CHAMBER, AND USE OF PHOTOTROPHIC MICRO-ORGANISMS IN THE RECOVERY OF HYDROGEN
PROCÉDÉ SERVANT À EXTRAIRE PAR UNE RÉACTION BIOLOGIQUE DE L'OXYGÈNE PRODUIT D'UN ESPACE DE RÉACTION ET UTILISATION DE MICROORGANISMES PHOTOTROPHES LORS DE L'OBTENTION D'HYDROGÈNE

(30) Priorität: 06.03.2017 DE 102017104648
(43) Veröffentlichungstag der Anmeldung: 15.01.2020
(73) Patentinhaber: Helmholtz-Zentrum für Umweltforschung GmbH - UFZ, 04318 Leipzig (DE)
(72) Erfinder: SCHMID, Andreas, 04105 Leipzig (DE); HOSCHEK, Anna, 67105 Schifferstadt (DE); BÜHLER, Bruno, 04159 Leipzig (DE)
(74) Vertreter: Gulde & Partner
(86) Internationale Anmeldenummer: PCT/EP2018/055449
(87) Internationale Veröffentlichungsnummer: WO 2018/162465

(56) Entgegenhaltungen:
- DE-A1- 2 807 069
- DE-A1-102007 002 009
- DE-A1-102010 015 807
- RAINER GROSS ET AL: "Engineered catalytic biofilms for continuous large scale production of n -octanol and ( S )-styrene oxide", BIOTECHNOLOGY AND BIOENGINEERING, Bd. 110, Nr. 2, 1. Februar 2013 (2013-02-01), Seiten 424-436, XP55490337, US ISSN: 0006-3592, DOI: 10.1002/bit.24629
- NAGARAJAN DILLIRANI ET AL: "Recent insights into biohydrogen production by microalgae - From biophotolysis to dark fermentation", BIORESOURCE TECHNOLOGY, ELSEVIER, AMSTERDAM, NL, Bd. 227, 30. Dezember 2016 (2016-12-30), Seiten 373-387, XP029881857, ISSN: 0960-8524, DOI: 10.1016/J.BIORTECH.2016.12.104
- NADINE LADKAU ET AL: "The microbial cell-functional unit for energy dependent multistep biocatalysis", CURRENT OPINION IN BIOTECHNOLOGY., Bd. 30, 1. Dezember 2014 (2014-12-01), Seiten 178-189, XP055490341, GB ISSN: 0958-1669, DOI: 10.1016/j.copbio.2014.06.003
- ANNA HOSCHEK ET AL: "Overcoming the Gas-Liquid Mass Transfer of Oxygen by Coupling Photosynthetic Water Oxidation with Biocatalytic Oxyfunctionalization", ANGEWANDTE CHEMIE INTERNATIONAL EDITION, Bd. 56, Nr. 47, 20. November 2017 (2017-11-20), Seiten 15146-15149, XP055490345, ISSN: 1433-7851, DOI: 10.1002/anie.201706886

### SEQUENCE LISTING

<110> Helmholtz Zentrum für Umweltforschung GmbH - UFZ
<120> Verfahren zur bioreaktiven Extraktion erzeugten Sauerstoffs aus einem Reaktionsraum, sowie Verwendung von phototrophen Mikroorganismen bei der Gewinnung von Wasserstoff
<130> P15840WO-TAU/GL/ge
<160> 13
<170> BiSSAP 1.3.6
<210> 1
   <211> 76
   <212> DNA
   <213> Artificial Sequence
<220>
   <223> Primer
<400> 1
   tccggctcgt ataatgtgtg gaattgtgag cggataacaa tttcacacat actagtacca 60
   ggcatcaaat aaaacg 76
<210> 2
   <211> 19
   <212> DNA
   <213> Artificial Sequence
<220>
   <223> Primer
<400> 2
   tataaacgca gaaaggccc 19
<210> 3
   <211> 63
   <212> DNA
   <213> Artificial Sequence
<220>
   <223> Primer
<400> 3
   tgatttctgg aattcgcggc cgctttctag attgacaatt aatcatccgg ctcgtataat 60
   gtg 63
<210> 4
   <211> 50
   <212> DNA
   <213> Artificial Sequence
<220>
   <223> Primer
<400> 4
   acaccttgcc cgtttttttg ccggactgca gtataaacgc agaaaggccc 50
<210> 5
   <211> 18
   <212> DNA
   <213> Artificial Sequence
<220>
   <223> Primer
<400> 5
   cttttcctcg tagagcac 18
<210> 6
   <211> 68
   <212> DNA
   <213> Artificial Sequence
<220>
   <223> Primer
<400> 6
   gagccacccg cagttcgaaa aatagtacta gagtagtgga ggttactaga tggcaatcgt 60
   tgttgttg 68
<210> 7
   <211> 21
   <212> DNA
   <213> Artificial Sequence
<220>
   <223> Primer
<400> 7
   atcaggtaat tttatactcc c 21
<210> 8
   <211> 51
   <212> DNA
   <213> Artificial Sequence
<220>
   <223> Primer
<400> 8
   ctatttttcg aactgcgggt ggctccaagc gctcttttcc tcgtagagca c 51
<210> 9
   <211> 79
   <212> DNA
   <213> Artificial Sequence
<220>
   <223> Primer
<400> 9
   ctttcgtttt atttgatgcc tggtactatt tttcgaactg cgggtggctc caagcgctat 60
   caggtaattt tatactccc 79
<210> 10
   <211> 50
   <212> DNA
   <213> Artificial Sequence
<220>
   <223> Primer
<400> 10
   gggaggtatt ggaccgcatt gaactctagt atataaacgc agaaaggccc 50
<210> 11
   <211> 50
   <212> DNA
   <213> Artificial Sequence
<220>
   <223> Primer
<400> 11
   acgagccgga tgattaattg tcaatctaga gccaggcatc aaataaaacg 50
<210> 12
   <211> 19
   <212> DNA
   <213> Artificial Sequence
<220>
   <223> Primer
<400> 12
   ccatcaaaca ggattttcg 19
<210> 13
   <211> 20
   <212> DNA
   <213> Artificial Sequence
<220>
   <223> Primer
<400> 13
   tgccacctga cgtctaagaa 20

## Beschreibung

Die Erfindung betrifft ein Verfahren zur bioreaktiven Extraktion erzeugten Sauerstoffs aus einem Reaktionsraum.

### Stand der Technik

Wasserstoff gilt als Energieträger der Zukunft, da er einerseits eine hohe Energiedichte aufweist und andererseits eine umweltverträgliche Energiequelle darstellt. Er steht in unbegrenzten Mengen zu Verfügung und lässt sich durch verschiedene Verfahren aus Wasser gewinnen. Wasserstoff kann in einer Brennstoffzelle wieder in Strom und Wärme zurück gewandelt werden, als Endprodukt entsteht wiederum lediglich Wasser. Mittels Wasserstoff lassen sich zumindest theoretisch riesige Energiemengen auch langfristig zwischenspeichern.

Zur Herstellung von Wasserstoff sind insbesondere die Elektrolyse und die photokatalytische Wasserspaltung zu nennen.

Die Elektrolyse ist eine altbekannte und bewährte Methode zur Erzeugung von Wasserstoff. Mit Hilfe elektrischen Stroms wird Wasser in Wasserstoff und Sauerstoff zerlegt. Diese Methode ist zurzeit nur dann wirtschaftlich, wenn genügend günstiger Strom zur Verfügung steht.

Die photokatalytische Wasserspaltung - die Spaltung von Wasser in Wasserstoff und Sauerstoff mittels Sonnenlicht - stellt eine technologisch wichtige Alternative zur energieaufwendigen elektrolytischen Wasserspaltung dar. In diesem Fall werden in der Regel Algen oder Cyanobakterien eingesetzt, welche unter bestimmten Bedingungen während des Stoffwechselprozesses Wasserstoff an die Umgebung abgegeben können. So kann man in Algenreaktoren mittels Photosynthese Sonnenlicht und Wasser direkt in Wasserstoff umwandeln. Der enzymatische Prozess besteht aus zwei Schritten. Durch die photosynthetische Lichtreaktion erfolgt im ersten Schritt eine Aufteilung des Wassers in Protonen, Elektronen und molekularen Sauerstoff. Durch zum Beispiel Hydrogenasen werden die Protonen im zweiten Schritt zu molekularem Wasserstoff reduziert. Bei dieser oxygenen, das heißt Sauerstoff-freisetzenden Photosynthese, arbeiten zwei große, Cofaktorhaltige Proteinkomplexe, das Photosystem I (PSI) und das Photosystem II (PSII) zusammen. Das PSII spaltet mit Hilfe von Lichtenergie Wasser in Protonen, Sauerstoff und Elektronen und transferiert letztere über eine Elektronentransportkette auf das PSI.

Die Kopplung der Hydrogenase mit dem Photosystem I, sowie dem Wasser-spaltenden Photosystem II der Mikroorganismen ermöglicht demzufolge die Herstellung von Wasserstoff aus Licht und Wasser, bei der lediglich Sauerstoff als "Abfallprodukt" anfällt. Bei dem Aufeinandertreffen von Sauerstoff und Wasserstoff besteht durch die Bildung von Knallgas eine Explosionsgefahr. Zudem sind bekannte Hydrogenasen sauerstoffempfindlich. Es ist deshalb zunächst erforderlich, die Gase nach der Bildung schnell zu trennen, um die Explosionsgefahr (Entstehung von Knallgas) und die Degeneration der Hydrogenasen zu verringern. Das erfolgt zum Beispiel unter Verwendung Sauerstoff-resistenter Hydrogenasen (US 20090263846 A1), poröser Membranen (DE 102007002009 A1) oder zeitlicher Trennung von Sauerstoffbildung und Wasserstoffproduktion (US 4532210 A). So wird in DE 28 07 069 A1 ein Verfahren für die Herstellung von Wasserstoff mittels phototropher Mikroorganismen vorgeschlagen, bei welchem der entstehende Sauerstoff durch die Zugabe eines separeten Sauerstoff-bindenden Systems, beispielsweise eines Enzymsystems, gebunden wird.

Diese Techniken besitzen wesentliche Nachteile, so unterdrücken die Sauerstoff-resistenten Hydrogenasen nicht die Bildung von Knallgas, die Membrantechnologie verhindert nicht die Inaktivierung eines Sauerstoff-sensitiven Enzymsystems und durch zeitliche Trennung von Sauerstoffbildung und Wasserstoffproduktion wird das Verfahren in eine produktive und nichtproduktive Phase geteilt. Demnach ist die photokatalytische Wasserstoffproduktion in großtechnischem Ausmaß an spezifische Bedingungen gebunden, die produktionstechnisch nicht oder nur unter hohem Kostenaufwand realisierbar sind.

Die Aufgabe der Erfindung besteht deshalb darin, ein Verfahren zur Wasserstoffproduktion bereitzustellen, das unerwünscht entstehenden molekularen Sauerstoff von der chemischen Produktion eines Zielprodukts trennt und die Nachteile des Standes der Technik, wie Explosionsgefahr durch Knallgasbildung, zwei Prozessphasen und Inaktivierung von Sauerstoff-sensitiven Enzymen, vermeidet. Insbesondere besteht die Aufgabe darin, umweltfreundliche, preiswerte und stabile Photokatalysatoren zu finden, die in der Lage sind, photosynthetisch entstehenden Sauerstoff während einer Wasserstoffproduktion zu binden.

Die Aufgabe wird erfindungsgemäß gelöst durch ein Verfahren gemäß einem der Ansprüche. Das erfindungsgemäße Verfahren zur bioreaktiven Extraktion photokatalytisch erzeugten Sauerstoffs in einem Reaktionsraum, umfasst die folgenden Schritte:
- Bestrahlen zumindest eines phototrophen Mikroorganismus mit Licht unter anaeroben Bedingungen in einem Reaktionsraum, und
- in situ Binden von entstehendem Sauerstoff durch den Mikroorganismus durch Oxidation eines Sauerstoff-verwertenden Substrats, wobei der zumindest eine phototrophe Mikroorganismus zumindest ein Sauerstoff-umsetzendes Enzym aufweist oder produziert.

Das erfindungsgemäße Verfahren überwindet technische Herausforderungen, die aufgrund von sauerstoffsensiblen Prozessen bzw. Edukten, Produkten und Enzymen entstehen. So können auch in sauerstoffproduzierenden enzymatischen Verfahren sauerstoffsensible Mikroorganismen und Enzyme eingesetzt werden, ohne dass die Leistungsfähigkeit beeinträchtigt wird. Prozesse, in denen sowohl Wasserstoff als auch Sauerstoff anfallen und die somit potenziell Knallgas produzieren, werden entschärft, da beide Reaktionspartner in situ getrennt werden.

Dies ist möglich, indem der Sauerstoff direkt am Ort seiner Entstehung eingefangen und enzymatisch gebunden wird. Damit ist der entstehende Sauerstoff direkt am Ort seiner Entstehung deaktiviert. Die Deaktivierung erfolgt beispielsweise durch die Oxidation eines ebenfalls im Reaktionsraum vorhandenen Substrats in Form einer chemischen Bindung des Sauerstoffs an das Substrat, aufgrund derer der Sauerstoff nicht wieder in den Reaktionsraum entlassen wird. Der Vorteil besteht daher insbesondere darin, dass der Sauerstoff nicht nur separiert oder maskiert ist, sondern chemisch deaktiviert ist. Somit weist der Reaktionsraum und insbesondere der Mikroorganismus bevorzugt ferner ein Sauerstoff verwertendes Substrat auf.

Darüber hinaus ermöglicht die Erfindung, die Sauerstoffdeaktivierung quantitativ zu regeln indem die sauerstoffverbrauchende Reaktion gesteuert wird. Somit kann ein zuvor definierter Betrag an Sauerstoff dem Reaktionsraum zur Verfügung gestellt werden, der im Bereich von 0 (entstehender Sauerstoff vollständig gebunden) bis 1 (kein Sauerstoff gebunden) liegt.

Durch geeignete Wahl des Substrats kann ferner ein oxidiertes Zielprodukt hergestellt werden, so dass die Bindung des im eigentlichen Zielprozess entstehenden störenden Sauerstoffs zu einem weiteren Wertschöpfungsschritt führt.

Vorliegend handelt es sich bei dem entstehenden Sauerstoff um molekular vorliegenden Sauerstoff.

Erfindungsgemäß weist der Mikroorganismus ein sauerstoffumsetzendes Enzym auf oder produziert ein solches.

In weiter bevorzugter Ausführungsform ist das bevorzugte Zielprodukt Wasserstoff beziehungsweise Protonen, welche anschließend mit Elektronen zu Wasserstoffmolekülen reduziert werden. Alternativ ist das Zielprodukt Methanol, welches durch Reduktion von CO₂ mit den aus der Wasserspaltung stammenden Elektronen erzeugt wird. In beiden Fällen handelt es sich um einen Brennstoff, der beispielsweise als Ausgangsprodukt für eine Brennstoffzellenreaktion eingesetzt wird. Diese Reaktionen werden beispielsweise durch drei Dehydrogenasen, welche zu der Klasse der Oxidoreduktasen gehören, katalysiert.

CO₂ +2e⁻ → Formiat (I)

Formiat+ 2e⁻ → Formaldehyde (II)

Formaledhyde + 2e⁻ → Methanol (III)

Insbesondere weist der zumindest eine phototrophe Mikroorganismus daher ein Enzym ausgewählt aus der Gruppe Hydrogenase, Nitrogenase und/oder Oxidoreduktase auf.

Das Verfahren wird vorzugsweise in einem flüssigen, insbesondere wässrigen Medium durchgeführt. In bevorzugter Ausführungsform ist vorgesehen, in einem Prozess störenden Sauerstoff (O₂) aus dem Reaktionsraum zu entfernen, der bei der photosynthetischen Lichtreaktion aus Wasser oder wässrigen Flüssigkeiten oder Lösungen unter Verwendung phototropher Mikroorganismen im Reaktionsraum entsteht. Dabei weist der phototrophe Mikroorganismus, mindestens ein sauerstoffumsetzendes Enzym auf oder produziert dieses. Gleichzeitig werden sauerstoffverwertende Substrate eingesetzt.

Vorteilhafterweise weist der Mikroorganismus neben der Hydrogenase, Nitrogenase und/oder Oxidoreduktase zumindest ein weiteres, sauerstoffumsetzendes Enzym auf. Insbesondere bei einer Kombination von Hydrogenase und einer Oxidoreduktase findet der Wasserstofferzeugende und der Sauerstoffumsetzende Prozessschritt im gleichen Mikroorganismus und damit ohne räumliche Trennung statt. Der Sauerstoff wird direkt nach dessen Produktion umgesetzt und erfindungsgemäß deaktiviert. In bevorzugter Ausführungsform ist vorgesehen, dass das weitere Enzym eine Oxidoreduktase, insbesondere Oxidase und Oxygenase, ist.

Alternativ liegt im Reaktionsraum ein weiterer Mikroorganismus vor, der das weitere Enzym aufweist. Mit anderen Worten, weist bevorzugt ein Mikroorganismus Hydrogenase, Nitrogenase und/oder Oxidoreduktase als Wasserstoff-produzierendes Enzym und zusätzlich Oxidoreduktase als Sauerstoff-umsetzendes Enzym auf, oder zwei verschiede im Reaktionsraum vorhandene Mikroorganismen weisen je eines dieser Enzyme auf. Vorliegend wird nur eine Kombination aus den genannten Enzymen explizit erwähnt, wobei auch sämtliche andere Kombinationen ausdrücklich vorgesehen sind. In der Ausführungsform verschiedener Mikroorganismen finden der sauerstofferzeugende und der sauerstoffumsetzende Prozess nicht im selben Mikroorganismus, aber dennoch im selben Reaktionsraum statt. Der Vorteil besteht insbesondere darin, dass der sauerstoffumsetzende Prozess und damit das quantitative Maß an im Prozess vorhandenem Sauerstoff durch ein quantitatives Verhältnis der Mikroorganismen zueinander gesteuert werden kann.

Ferner ist bevorzugt, dass der Mikroorganismus selbst das Zielprodukt, also beispielsweise Wasserstoff oder Methanol, erzeugt. Mit anderen Worten, es wird in einer bevorzugten Ausführungsform des erfindungsgemäßen Verfahrens bei der Bestrahlung des zumindest einen phototrophen Mikroorganismus neben Sauerstoff auch Wasserstoff oder Methanol frei.

Somit sieht das erfindungsgemäße Verfahren in einer bevorzugten Ausführungsform vor, dass mindestens ein phototropher Mikroorganismus unter Lichteinwirkung mit Wasser oder einer wässrigen Flüssigkeit oder Lösung in Kontakt gebracht wird, wobei ein Mikroorganismus gewählt wird, der neben den Hydrogenasen weiterhin Oxidoreduktasen als Sauerstoff-umsetzende Enzyme aufweist oder produziert oder ein weiterer Mikroorganismus im gleichen Reaktionsraum gewählt wird, der Oxidoreduktasen als Sauerstoff-umsetzende Enzyme aufweist oder produziert.

Die Oxidoreduktase ist vorzugsweise ausgewählt aus der Gruppe der Oxidasen oder Oxygenasen, vorzugsweise Monooxygenasen, Dioxygenasen oder coenzym-unabhängige Oxygenasen. Eine Monooxygenase als Sauerstoff verbrauchendes Enzym sollte das Enzym der Wahl sein, wenn das stöchiometrische Gleichgewicht im Reaktionsraum von Interesse ist. Es ergibt sich folgendes Reaktionssystem:

Die photosynthetische Lichtreaktion: 2 H₂O → 4 H⁺ + O₂ +4e⁻ (IV)

Reaktion der Monooxygenase: S + O₂ +2e⁻ + 2 H⁺ → S_{oxidiert} + H₂O (V)

Reaktion der Hydrogenase : 2 H⁺ + 2 e⁻ → H₂ (VI)

Besonders bevorzugt sind Oxidoreduktasen vom AlkB-Typ, die in der Literatur als Katalysatoren zur Herstellung überwiegend von weniger stark oxidierten Produkten bekannt sind. Diese eignen sich besonders um aus Alkanen und deren Carbonsäuren bzw. Carbonsäureestern, überwiegend Produkte höherer Oxidationsstufen herzustellen. - Derartige Oxidoreduktasen sind ferner zur selektiven Oxidation von Alkanen und deren Carbonsäuren bzw. -estern befähigt, wobei zu erwartende Nebenprodukte, insbesondere an anderen als endständigen Kohlenstoffatomen oxidierte Alkane, nur in unerwartet geringem Ausmaß oder überhaupt nicht in nachweisbaren Mengen herzustellen.

Vorliegend werden die Substrate vorzugsweise unter Verwendung einer Oxidoreduktase vom AlkB-Typ in Anwesenheit von Sauerstoff oxidiert. AlkB stellt eine zunächst aus dem AlkBGT-System aus *Pseudomonas putida Gpo1* bekannt gewordene Oxidoreduktase dar, die von zwei weiteren Polypeptiden, AlkG und AlkT, abhängig ist. AlkT wird als FAD abhängige Rubredoxin-Reduktase charakterisiert, die Elektronen aus NADH an AlkG weitergibt. Bei AlkG handelt es sich um ein Rubredoxin, ein eisenhaltiges Redoxprotein, das als direkter Elektronendonor für AlkB fungiert. In einer bevorzugten Ausführungsform wird unter demselben Begriff "Oxidoreduktase des alkB-Typs" ein Polypeptid mit einer Sequenzhomologie von zunehmend bevorzugt wenigstens 75, 80, 85, 90, 92, 94, 96, 98 oder 99 % zur Sequenz des AlkB von *Pseudomonas putida Gpo1* (Datenbankcode: CAB54050.1; dieser Datenbankcode stammt aus dem Stand der Technik, nämlich aus der NCBI Datenbank, genauer dem am 15. November 2011 online verfügbaren Release) mit der Fähigkeit, die erfindungsgemäßen Substrate, wie Alkane und deren Carbonsäuren zu oxidieren. In einer besonders bevorzugten Ausführungsform handelt es sich bei der Oxidoreduktase vom AlkB-Typ um eine mit den AlkG (CAB54052.1)- und AlkT (CAB54063.1)-Polypeptiden aus *Pseudomonas putida Gpo1* funktionell zusammenwirkende, Alkane oxidierende Oxidoreduktase. In einer bevorzugtesten Ausführungsform handelt es sich bei der Oxidoreduktase vom AlkB-Typ um AlkB aus dem AlkBGT-System aus *Pseudomonas putida Gpo1* oder eine Variante davon.

Mit besonderem Vorteil werden Substrate eingesetzt, die nach der Oxidation als Edukte weiterverwendet werden und sogenannte *value added products* für das erfindungsgemäße Verfahren bilden oder aber solche, die im Reaktionsraum unerwünscht sind und durch die Oxidation deaktiviert werden. Somit ist neben der Produktion des Zielprodukts, wie Wasserstoff oder Methanol, auch die Sauerstoffumsetzung ein Zielprozess des Verfahrens. Es handelt sich dann nicht mehr nur um die Bindung von unerwünschtem Sauerstoff, der die Produktion des ersten Zielprodukts stört, sondern um die Bindung von entstehendem Sauerstoff zu einem zweiten Zielprodukt. Ein Einsatzbeispiel, in dem ein unerwünschtes Edukt durch das erfindungsgemäße Verfahren durch Oxidation deaktiviert wird ist die Schmutzwasseraufbereitung. Hier liegen verschiedene Substrate als unerwünschte Stoffe im Schmutzwasser vor. Das Schmutzwasser wird durch Oxidation dieser Substrate aufgereinigt. Je nach eingesetztem Mikroorganismus wird dabei als erwünschtes Nebenprodukt beispielsweise Wasserstoff als Brennstoff produziert.

In bevorzugter Ausführungsform fungieren im erfindungsgemäßen Verfahren Kohlenstoffverbindungen als Substrate, die zugesetzt werden oder die sich in der wässrigen Ausgangslösung befinden. Dabei handelt es sich vorzugsweise um Carbonsäuren oder Carbonsäureester mittelkettiger Alkane mit einer C₃-C₂₀, insbesondere C₅-C₁₅, vorzugsweise C₇-C₁₀ -Kette. Ein besonders bevorzugtes Beispiel ist Methylnonanoat (C₈H₁₇-COOH-CH₃)-Das erfindungsgemäße Verfahren sieht bevorzugt vor, dass als phototropher Mikroorganismus Algen, Purpurbakterien oder Cyanobakterien eingesetzt werden, wobei Cyanobakterien bevorzugt sind. Diese betreiben Photosynthese und weisen sauerstoffumsetzende Enzyme auf, produzieren diese oder sind in geeignetem Maße zur genetischen Modifizierung geeignet, um derartige Enzyme aufzuweisen oder zu produzieren. Mit besonderem Vorteil wird als Mikroorganismus ein, insbesondere genetisch modifizierter, Cyanobakterienstamm eingesetzt, der das Alkan-Monooxygenase-Enzymsystem AlkBGT aufweist.

In besonders bevorzugter Ausgestaltung des erfindungsgemäßen Verfahrens wird der genetisch modifizierte Cyanobakterienstamm *Synechocystis sp.* PCC6803 als Mikroorganismus eingesetzt. Dieser weist das Alkan-Monooxygenase-Enzymsystem AlkBGT auf.

Somit betrifft ein weiterer Aspekt der Anmeldung eine Cyanobakterienzelle umfassend eine Hydrogenase und die von *alkBGT* kodierte Alkanmonooxygenase der SEQ ID NO: 1 *(aus Pseudomonas putida GPo1)* oder einer Variante davon, insbesondere einem Enzym, das zu mindestens 80% mit der SEQ ID NO: 1 identisch ist.

Die Lehre der vorliegenden Erfindung kann nicht nur unter Verwendung der exakten Aminosäure- oder Nukleinsäuresequenzen der hierin beschriebenen biologischen Makromoleküle ausgeführt werden, sondern auch unter Verwendung von Varianten derartiger Makromoleküle, die durch Deletion, Addition oder Substitution einer oder mehr als einer Aminosäure oder Nukleinsäure erhalten werden können.

In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologen" gebraucht, wie hierin verwendet, eine andere Nukleinsäure- oder Aminosäuresequenz, die mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder letztere lediglich konservativ substituiert sind, beispielsweise ein Glutamat statt einem Aspartat oder ein Leucin statt einem Valin. Es besteht keine Notwendigkeit, dass die Sequenz über ihre gesamte Länge eine entsprechend hohe Homologie aufweist, erfindungsgemäß können auch Fusionsproteine oder für solche kodierende Nukleinsäuren verwendet werden, die einen Teil mit entsprechender Homologie und/oder Aktivität aufweist. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd editi*on.*

In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatisch Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den KM- und/oder kcat-Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz.

Insbesondere in vorgenannter Ausführungsform wird als Substrat Methylnonanoat verwendet.

Ein weiterer Aspekt der Anmeldung betrifft die Verwendung von phototrophen Mikroorganismen, die neben Hydrogenasen Oxidoreduktasen als Sauerstoff-umsetzende Enzyme aufweisen oder produzieren, bei der Gewinnung von Wasserstoff (H₂) und Sauerstoff (O₂) aus Wasser oder wässrigen Flüssigkeiten und Lösungen und zur simultanen in-situ Entfernung des Sauerstoffs (O₂) unter Verwendung entsprechender Substrate, die mit dem Sauerstoff Produkte bilden. Bevorzugt betrifft die Erfindung die Verwendung von phototrophen Mikroorganismen, die neben Hydrogenasen Oxidoreduktasen als Sauerstoffumsetzende Enzyme aufweisen oder produzieren in dem erfindungsgemäßen Verfahren. Insbesondere bevorzugt ist die Verwendung des erfindungsgemäßen Verfahrens bei der Schmutzwasseraufbereitung. Hierbei wird ein Gemisch undefinierter organischer Substanzen, die im Sinne der Erfindung als Substrate wirken, für die sauerstoffverwertende Reaktion des Mikroorganismus zur Verfügung gestellt. Die Wahl einer nicht spezifischen, nichtselektiven Oxygenierungsreaktion ermöglicht die Oxidation und damit die Inaktivierung der im Abwasser auftretenden Schadstoffe. Dabei findet vorteilhafterweise gleichzeitig eine Erzeugung von Wasserstoffgas als Quelle für Energieerzeugung und eine Oxidation von gelöstem organischen Kohlenstoff im Abwasser statt.

Als Mikroorganismus wird vorzugsweise ein Algenstamm, ein Purpurbakterienstamm oder ein Cyanobakterienstamm verwendet. Besonders bevorzugt wird ein Stamm, der das Alkan-Monooxygenase-Enzymsystem AlkBGT aufweist, wie beispielsweise *Synechocystis sp. PCC6803,* eingesetzt.

Das erfindungsgemäße Verfahren wird bevorzugt im Photobioreaktor durchgeführt. Somit betrifft ein weiterer Aspekt der Anmeldung einen Photobioreaktor zur Ausführung des erfindungsgemäßen Verfahrens, insbesondere umfassend den genetisch modifizierten Cyanobakterienstamm *Synechocystis sp.* PCC6803, der das Alkan-Monooxygenase-Enzymsystem AlkBGT aufweist oder produziert. Hierbei liegt der Mikroorganismus, insbesondere der Bakterienstamm als Biofilm vor.

Photobioreaktoren sind Fermenter, in denen die phototrophen Mikroorganismen zum Beispiel Algen, Cyanobakterien und Purpurbakterien kultiviert werden, in denen also entweder das Wachstum und die Vermehrung dieser Zellen ermöglicht wird oder die Produktion unterschiedlicher Substanzen mittels phototropher Zellen gefördert wird. Im Unterschied zu konventionellen biotechnischen Fermentationsprozessen sind phototrophe Vorgänge lichtabhängig. Um den Zellen einen aktiven Stoffwechsel zu ermöglichen, werden sie möglichst optimal sowohl mit Licht als auch mit verschiedenen Nährlösungen bzw. gelösten Substraten versorgt.

Die Photobioreaktoren variieren erheblich in Material und Konstruktionsweise. Ihnen allen gemein ist, dass sie auf Basis ihrer Konstruktion eine optimale Lichtversorgung der Mikroorganismen gewährleisten sollen. Auf dem Markt befinden sich zum Beispiel Reaktoren, die mit Kunststoff-Beuteln oder Schläuchen arbeiten oder Systeme die mit flachen Behältern oder Röhren die Biomasse in dünnen Schichten der Sonne exponieren. Jeder Reaktor hat neben dem lichtdurchlässigen Photosyntheseteil auch einen Zulauf für CO₂ und Substrate und eine Einrichtung die Mikroorganismen zu ernten. Meist wird der Zustand und die Dichte der Kultur noch über eine sensible Mess-Elektronik detektiert, um regelnd eingreifen zu können.

Der Röhrenreaktor ist ein ausgereifter und bereits in Großanlagen verbauter Photobioreaktor. Die Mikroorganismensuspension wird in durchsichtigen Röhren aus Glas oder Plastik geführt und mittels einer Pumpe in Bewegung gehalten.

Der Helix- oder Coil-Reaktor ist im Grunde ein Röhrenreaktor, der allerdings keine Glasröhren einsetzt, sondern einen flexiblen Schlauch. Dieser wird dann kreisförmig angeordnet, die Prozessführung ähnelt dem Röhrenreaktor.

Der Beutel- oder Schlauch-Reaktor ist ein Photobioreaktor auf Basis von einzelnen Kompartimenten aus Kunststofffolie, oft hängend angeordnet. Es gibt hier auch Ansätze funktionale Kunststoffe einzusetzen und Ideen, die Beutel auf dem Wasser zu positionieren. Die Durchmischung erfolgt meist durch den CO₂- Eintrag und ist damit relativ energieeffizient. Die Folie ist dabei nicht auf einen langfristigen Einsatz ausgerichtet, sondern wird von Zeit zu Zeit erneuert.

Beim Kessel-Reaktor wird ein üblicherweise säulenförmiger Flüssigkeitsbehälter eingesetzt, in dem die Algenbiomasse gezüchtet wird. Je nach Design des Behälters, wird über verschiedene Systeme Licht in den Behälter eingebracht. Dies kann Sonnenlicht sein, oder artifizielle Lichtquellen. Manche Ansätze nutzen dazu Linsensysteme und Lichtleiter. Eine Durchmischung kann über verschiedene Mechanismen erfolgen, wie zum Beispiel Gaseintrag oder Umwälzung durch Pumpen.

Im Flachplatten-Reaktor werden die Mikroorganismen dabei im Kulturmedium in flachen Schichten dem Licht ausgesetzt. Begrenzt wird der Reaktor durch Glas- oder Plastik-Platten, die Durchmischung erfolgt durch den Gaseintrag.

Im Dünnschicht-Reaktor, wird das Prinzip der Minimierung der Mikroorganismenschicht zur optimalen Lichtausbeute konsequent weiter verfolgt. Damit soll die Selbst-Überschattung dichterer Suspensionen gemindert werden. Immobilisierte Mikroorganismen werden dabei auf einem Trägermaterial dem Licht exponiert.

Beim erfindungsgemäßen Verfahren werden bevorzugt Röhrenreaktoren eingesetzt.

Zusammenfassend ermöglicht die Erfindung eine Kombinierung zweier bislang unabhängiger Techniken, die photokatalytische Wasserstoffgasproduktion und die photokatalytische Sauerstoffreaktion. Simultan zur Herstellung von Wasserstoff als Biotreibstoff wird dabei ein Substrat oxidiert und dabei deaktiviert oder ein Mehrwertprodukt hergestellt.

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.

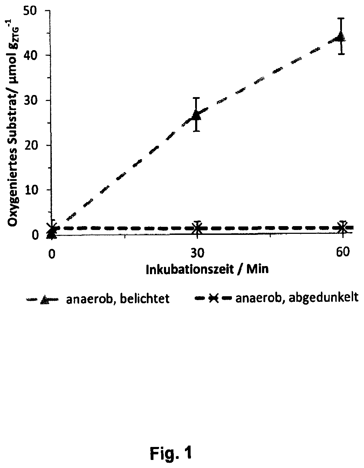

- **Figur 1****:** graphische Darstellung einer Extraktion von photosynthetisch erzeugtem Sauerstoff durch das Alkan-Monooxygenase Enzymsystem AlkBGT, welches genetisch in den Cyanobakterien-Stamm *Synechocystis sp.* PCC6803 eingeführt wurde, in erfindungsgemäßer Durchführung.

Zur Durchführung des erfindungsgemäßen Verfahrens kommen verschiedene Bedingungen in Frage. Essentiell ist dabei lediglich die Anwesenheit von molekularem Sauerstoff als Oxidationsmittel.

### Beispiel 1:

### Herstellung eines genetisch modifizierten Stamms - Synechocystis sp. PCC6803, enthaltend das Alkan-Monooxygenase Enzymsystem AlkBGT

Die Einführung der Gene kodierend für das Alkan-Monooxygenase Enzymsystem AlkBGT erfolgte über einen Plasmid-basierten Ansatz (pRSF_Ptrc1O:BGTII). Die folgenden Protokolle und Klonierungsschritte beschreiben die Konstruktion des Plasmides. In Tabelle 1 sind die Stämme und Plasmide aufgeführt, welche während des Klonierungs-Verfahrens verwendet und erzeugt wurden.

**Tabelle 1: Stämme/ Plasmid-Konstruktionen die während des Klonierungs-Verfahrens verwendet und erzeugt wurden.**

| Stamm/ Plasmid | Beschreibung | Referenz |
|---|---|---|
| *E. coli* DH5α | F⁻ φ80/*ac*ZΔM 15 Δ(*lac*ZYA-*arg*F) U169 *rec*A1 *end*A1 *hsd*R17 (rK⁻, mK⁺) *pho*A *supE44* λB⁻ *thi*⁻¹ *gyr*A96 *rel*A1 | (Hanahan 1983) |
| *Synechocystis sp.* PCC6803 | Geographische Ursprung: Kalifornien, USA Erhalten von der Pasteur Culture Collection of Cyanobacteria (PCC, Paris, Frankreich) | (Stanier et al. 1971) |
| pBT10 | Alkane-Monooxygenase Expressionssystem (alkBFG, alkST) in pCOM10 | (Schrewe et al. 2011) |
| pSB1AC3_Ptrc1O:GFPmut3B | P_{trc1O} Promoter, GFPmut3B Gen (BBa_E0040) in pSB1AC3 | (Huang et al. 2010) |
| pSB1AC3_Ptrc1O:Term | pSB1AC3mit dem P_{trc1O} Promoter aus pSB1AC3_Ptrc1O:GFPmut3B via Xbal, Pstl (Gibson Klonierung) | diese Arbeit |
| pSB1AC3_PrnpB:lacl | PrnpB (konstitutiver Promoter des rnaseP Gens) kontrollierend eine Variante des lac Repressors lacl in pSB1AC3 | (Huang et al. 2010) |
| pSB1AC3_PrnpB:lacl_Ptrc1O:Te rm | pSB1AC3_Ptrc1O:Term mit PrnpB:lacl aus pSB1AC3_PrnpB:lacl via Xbal (Restriktion, Ligation) | diese Arbeit |
| pPMQAK1 | Broad host range Plasmid, RSF ori, mob Gene | (Huang et al. 2010) |
| pPMQAK1_PrnpB:lacl_Ptrc1O:T erm | pPMQAK1 mit PrnpB:lacl_Ptrc1O:Term aus pSB1AC3_PrnpB:lacl_Ptrc1O:Term via EcoRI, Pstl (Restriktion, Ligation) | diese Arbeit |
| pPMQAK1_PrnpB:lacl_Ptrc1O:B GTII | pPMQAK1_PrnpB:lacl_Ptrc1O:Term mit *alkBGT* Genen aus pBT10 (Gene aufeinander folgend , optimierte RBS, C-terminaler Strep-tag II) via Spel (Gibson Klonierung) | diese Arbeit |
| pRSF_Ptrc1O:BGTII | pPMQAK1_PrnpB:lacl_Ptrc1O:BGTII mit zusätzlichem Terminator (biobrick #BBa_B0015 via Xbal (Gibson Klonierung) | diese Arbeit |

Als Teil des Klonierungsverfahrens wurden die folgenden Verfahrensschritte wie Restriktion, Amplifizierung u.s.w. wie im Folgenden angegeben durchgeführt.

Die Restriktions-Endonukleasen wurden von Thermo Scientific - Germany GmbH (Schwerte, Deutschland) bezogen und entsprechend der Empfehlung eingesetzt.

Eine Amplifizierung von DNA Fragmenten wurde durch polymere Kettenreaktion (PCR) bei Anwendung der Phusion High Fidelity (HF) DNA Polymerase von Thermo Scientific - Germany GmbH (Schwerte, Deutschland) über das empfohlene 3-Schritt Protokoll mit entsprechenden Primern (aufgeführt in Tabelle 2) durchgeführt. Entsprechende Anlagerungs- Temperaturen (T_{An.}) und Elongations-Zeiten (t_{EI.}) sind im jeweiligen Verfahrensschritt unten beschrieben.

Eine gewünschte Overlap-Extension-PCR (OE-PCR) wurde durch Einsatz von je 50 ng der DNA Fragmente in einem 100 µL Standard PCR-Ansatz durchgeführt. Entsprechende Primer wurden nach 5 PCR Zyklen zugegeben.

Plasmid-DNA wurde mittels FastAP Thermosensitive Alkaline Phosphatase von Thermo Scientific - Germany GmbH (Schwerte, Deutschland) entsprechend der Empfehlung dephosphoryliert.

Anschließend erfolgte eine Aufreinigung von Plasmid-DNA und amplifizierter DNA-Fragmente über das PCR Clean-up Gel Extraktions-Kit von MACHERY-NAGEL GmbH & Co. KG (MACHEREY-NAGEL GmbH & Co. KG, Düren, Deutschland).

Gibson Klonierung erfolgte über eine einschrittige, isothermale, *in vitro* rekombinante Klonierungsmethode beschrieben durch Gibson et al. 2009 (Gibson et al. 2009).

Ligation erfolgte über die T4 DNA Ligase von Thermo Scientific - Germany GmbH (Schwerte, Deutschland) entsprechend der Empfehlung.

Schließlich erfolgte eine Verifizierung von PCR-amplifizierten DNA Sequenzen durch Sequenzierung über die Firma Eurofins MWG (Ebersberg, Deutschland).

Auf die in Tabelle 1 genannten bekannten Verfahren wird in der vorliegenden Arbeit Bezug mit der in Tabelle 1 vermerkten Referenz Bezug genommen.

**Tabelle 2: Primer die während des Klonierungs-Verfahrens eingesetzt wurden.**

| Primer# | SEQ.-No. | Sequenz | |
|---|---|---|---|
| PAH055 | 1 | | |
| PAH056 | 2 | TATAAACGCAGAAAGGCCC | |
| PAH057 | 3 | | |
| PAH058 | 4 | | |
| PAH069 | 5 | CTTTTCCTCGTAGAGCAC | |
| PAH070 | 6 | | |
| PAH071 | 7 | ATCAGGTAATTTTATACTCCC | |
| PAH072 | 8 | | |
| PAH073 | 9 | | |
| PAH077 | 10 | | |
| PAH078 | 11 | | |
| SPAH017 | 12 | CCATCAAACAGGATTTTCG | Klonierungs- |
| SPAH023 | 13 | TGCCACCTGACGTCTAAGAA | |

### Verfahren

### Konstruktion von pSB1AC3 Ptrc1O:Term

| | |
|---|---|
| Restriktion: | pSB1AC3_Ptrc1O:GFP (Xbal + Pstl) → pSB1AC3 (Xbal, Pstl) |
| Amplifizierung: | Ptrc1O:Term part I von pSB1AC3_Ptrc1O:GFP (PAH055 + PAH056 → 186 BP, T_{An}: 60°C, t_{Elong}: 10 sec) Ptrc1O:Term part II von part I (PAH057 + PAH058 → 262 BP, T_{An}: 72°C, t_{Elong}: 10 sec) |
| Gibson Klonierung: | pSB1AC3 (Xbal, Pstl) + Ptrc1O:Term part II → pSB1AC3 Ptrc1O:Term |

### Konstruktion von pSB1AC3 PrnpB:lacl Ptrc1O:Term

| | |
|---|---|
| Restriktion: | pSB1AC3_Ptrc1O:Term (Xbal) |
| Dephosphorylierung: | pSB1AC3_Ptrc1O:Term (Xbal) (FastAP, Thermo) |
| Restriktion: | pSB1AC_PrnpB:lacl (Xbal + Spel) |
| Ligation: | pSB1AC3_Ptrc1O:Term (Xbal) + PrnpB:lacl (Xbal_Spel) (1:2) → pSB1AC3 PrnpB:lacl Ptrc1O:Term |
| Verifizierung by PCR: | Klone mit dem PrnpB:lacl Fragment in gewünschter Richtung wurden |
| | durch PCR bestimmt (SPAH017 + SPAH023 → 500 BP, T_{An}: 61 °C, t_{Elong}: 15 sec). |

### Konstruktion von pPMQAK1 PrnpB:lacl Ptrc1O:Term

| | |
|---|---|
| Restriktion: | pPMQAK1 (EcoRI + Pstl) |
| Restriktion: | pSB1AC3_PrnpB:lacl_Ptrc1O:Term (EcoRI + Pstl) |
| Ligation: | pPMQAK1 (EcoRI, Pstl) + PrnpB:lacl_Ptrc1O:Term (EcoRI, Pstl) (1:5) → pPMQAK1 PrnpB:lacl Ptrc1O:Term |

### Konstruktion von pPMQAK1 PrnpB:lacl Ptrc1O:BGTII

| | |
|---|---|
| Restriktion: | pPMQAK1_PrnpB:lacl_Ptrc1O:Term (Spel) |
| Amplifizierung: | oAlkBII (PAH059 + PAH067 → 1283 BP, T_{An}: 65°C, t_{Elong}: 25 sec) oAlkGII (PAH068 + PAH069 → 568 BP, T_{An}: 60°C, t_{Elong}: 25 sec) oAlkTII (PAH070 + PAH071 → 1204 BP, T_{An}: 65°C, t_{Elong}: 25 sec) |
| OE-PCR: | oAlkBII+ oAlkGII (PAH063 + PAH072 → 1859 BP, T_{An}: 65°C, t_{Elong}: 60 sec) |
| | →oBGII oBGII + oAlkTII (PAH063 + PAH073 → 3096 BP,T_{An}: 57°C, t_{Elong}: 90sec) |
| | →oBGTII |
| Gibson: | pPMQAK1_PrnpB:lacl_Ptrc1O:Term (Spel) + oBGT → pPMQAK1 PrnpB:lacl Ptrc1O:BGTII |

### Konstruktion von pRSF Ptrc1O:BGTII

| | |
|---|---|
| Restriktion: | pPMQAK1_PrnpB:lacl_Ptrc1O:BGTII (Xbal) |
| Amplifizierung: | Term von pSB1AC3_Ptrc1O:GFP (PAH077 + PAH078 → 191 BP, T_{An}: 60°C, t_{Elong}: 5 sec) |
| Gibson: | pPMQAK1_PrnpB:lacl_Ptrc1O:oBGTII (Xbal) + Term → pRSF Ptrc1O:BGTII |

### Wachstumsbedingungen für Synechocystis sp. PCC6803

*Synechocystis sp.* PCC6803 wurde in YBG11 medium, basierend auf Shcolnick et al. 2007, bei Zugabe von 50 mM HEPES Puffer kultiviert (Shcolnick et al. 2007). Als Selektionsdruck wurden 50 µg mL⁻¹ Kanamycin zugesetzt. Standard Kultivierungsbedingungen umfassen ein Kulturvolumen von 20 mL YBG11 medium in 100 mL Erlenmeyer Schüttelflaschen mit Schikane, welche in einem Orbitalschüttler (Multitron Pro shaker, Infors, Bottmingen, Schweiz) bei 150 rpm (2.5 cm Amplitude) eingesetzt wurden. Die Kultivierungstemperatur betrug 30°C, bei einer Lichtintensität von 50 µmol m⁻² s⁻¹ (LED), 0.04 % CO₂ und einer Luftfeuchtigkeit von 75 %. Das Wachstum wurde über die optische Dichte bei einer Wellenlänge von 750 nm über ein Spektrophotometer (Libra S11, Biochrom Ltd, Cambridge, UK) verfolgt. Vorkulturen wurden über 200 µL einer Cryostock-Lösung innokuliert und unter Standard-Bedingungen für 4 - 6 Tage kultiviert. Hauptkulturen wurden ausgehend von dieser Vorkultur mit einer Start-OD₇₅₀ von 0.08 innokuliert und für 3 Tage unter Standard-Bedingungen kultiviert bis die Genexpression für einen weiteren Tag durch Zugabe von 2 mM IPTG induziert wurde.

YBG11: 1.49 g L⁻¹ NaNO₃, 0.074 g L⁻¹ MgSO₄ · 7 H₂O, 0.305 g L⁻¹ K₂HPO₄, 10 mL L⁻¹ YBG11 Spurenelemente (100x) , 0.019 g L⁻¹ Na₂CO₃, 50 mM HEPES (pH 7.2); YBG11 Spurenelemente (100x): 0.36 g L⁻¹ CaCl₂ · 2 H₂O, 0.63 g L⁻¹ Zitronensäure, 0.28 g L⁻¹ Borsäure, 0.11 g L⁻¹ MnCl₂ · 4 H₂O, 0.02 g L⁻¹ ZnSO₄ · 7 H₂O, 0.039 g L⁻¹ Na₂MoO₄ · 2 H₂O, 0.007 g L⁻¹ CuSO₄ · 5 H₂O, 0.003 g L-1 Co(NO₃)₂ · 6 H₂O, 0.1 g L⁻¹ FeCl₃ · 6 H₂O, 0.6 g L⁻¹ Na₂EDTA · 2 H₂O

### Transformation von Synechocystis sp. PCC6803 durch Elektroporation

Transformation von *Synechocystis sp.* PCC6803 mit dem Plasmid pRSF_Ptrc1O:BGTII wurde über Elektroporation, basierend auf einer Methode nach Ferreira et al. 2014, durchgeführt (Universidade do Porto Ferreira 2014). Elektrokompetente Zellen wurden ausgehend von einer 50 mL YBG11 Hauptkultur (in 100 mL Erlenmeyer Schüttelkolben mit Schikane) mit einer OD₇₅₀ von 0.5 - 1 hergestellt. Die Zellen wurden durch Zentrifugation geerntet (10 min, 3180 g, 4°C), dreimal mit je 10 mL HEPES Puffer (1 mM, pH 7.5) gewaschen und in 1 mL HEPES Puffer resuspendiert. Die elektrokompetenten Zellen wurde nach Zugabe von 5 % (v/v) DMSO bei - 80°C gelagert. Zur Elektroporation wurden 0.2 - 1.0 µg Plasmid-DNA zu 60 µL elektrokompetenten Zellen in eine Elektroporationsküvette (2 mm Elektrodenabstand) gegeben, bei 2500 V für 5 ms gepulst (12.5 kV cm⁻¹) (Eppendorf Eporator, Eppendorf Vertrieb Deutschland GmbH, Wesseling-Berzdorf, Deutschland) und anschließend in 50 mL YBG11 Medium (in 100 mL Erlenmeyer Schüttelkolben mit Schikane) transferiert. Nach Kultivierung unter Standard-Bedingungen für 24 h wurden die Zellen durch Zentrifugation geerntet (10 min, 3180 g, RT), in 100 µL YBG11 Medium resuspendiert und auf BG11 Agarplatten mit 50 µg mL⁻¹ Kanamycin ausgestrichen ((Stanier et al. 1971). Einzelne Kolonien wurden nach 4 - 6 Tagen bei 30°C, 20-50 µmol m⁻² s⁻¹ Lichtintensität (Leuchtstoffröhren), 0.04 % CO₂ und 80 % Luftfeuchtigkeit (poly klima GmbH, Freising, Deutschland) auf frische BG11 Agarplatten transferiert und erneut inkubiert. Die Zellmasse einer Agarplatte wurde schließlich zur Innokulation einer 20 mL YBG11 Vorkultur genutzt.

BG11 Agarplatten: 1.5 g L⁻¹ NaNO₃, 0.075 g L⁻¹ MgSO₄ · 7 H₂O, 0.036 g L⁻¹ CaCl₂ · 2 H₂O, 0.006 g L⁻¹ citric acid, 0.04 g L⁻¹ K₂HPO₄, 0.006 g L⁻¹ Eisen-Ammonium-Citrat, 0.001 g L⁻¹ Na₂EDTA, 0.02 g L⁻¹ Na₂CO₃, 1 mL L⁻¹ BG11 Spurenelemente (1000x), 0.3 % Na₂S₂O₃, 10 mM HEPES (pH 8), 1.5 % Agar; BG11 Spurenelemente (1000x) : 2.86 g L⁻¹ Borsäure, 1.8 g L⁻¹ MnCl₂ · 4 H₂O, 0.22 g L⁻¹ ZnSO₄ · 7 H₂O, 0.39 g L⁻¹ Na₂MoO₄ · 2 H₂O, 0.08 g L⁻¹ CuSO₄ · 5 H₂O, 0.05 g L⁻¹ Co(NO₃)₂ · 6 H₂O

Figur 1 zeigt eine Bio-reaktive Extraktion von photosynthetisch erzeugtem Sauerstoff durch das Alkan-Monooxygenase Enzymsystem AlkBGT, welches genetisch in den Cyanobakterien-Stamm *Synechocystis sp.* PCC6803 eingeführt wurde. Die photosynthetische Lichtreaktion wurde durch Beleuchtung (Lichtintensität von 50 µmol m⁻²s⁻¹) induziert, während die Kontroll-Reaktion ohne Beleuchtung stattfand. Als oxygeniertes Produkt wurde terminal hydroxyliertes Methyl-Nonanoat detektiert. CDW = Zelltrockengewicht.

Hierzu wurde das Alkan-Monooxygenase Enzymsystem AlkBGT in den Cyanobakterien-Stamm *Synechocystis sp.* PCC6803 eingesetzt (über das Plasmid pRSF_Ptrc1O:BGTII, siehe Herstellung des modifizierten Stamms oben), welcher ein potentieller Biokatalysator zur photosynthetischen Wasserstoff Produktion ist. Nach Umsetzung des Reaktionssystems von aeroben zu anaeroben Bedingungen und nach Zugabe von Methyl-Nonanoat als Substrat, konnte die Bildung von oxygeniertem Produkt bei Beleuchtung detektiert werden (Figur 1).

Da bei Reaktionsbedingungen in Dunkelheit keine Produktbildung beobachtet wurde, weisen die Ergebnisse nach, dass der für die Reaktion gebrauchte Sauerstoff aus der photosynthetischen Lichtreaktion stammte. Die spezifische Aktivität betrug 0,9 ± 0,1 µmol_{oxygeniertes Substrat} min⁻¹ g_{CDW}⁻¹ für die ersten 30 Minuten. Die Sauerstoff-Bildungsrate bei gleicher Belichtungs-Intensität von 50 µmol m⁻² s⁻¹ betrug 3,7 ± 0,5 µmol_{O2} min⁻¹ g_{CDW}⁻¹ (ohne Substrat-Zugabe). Somit konnten mit dem nicht-optimierten Biokatalysator bereits nahezu 25 % des entstandenen Sauerstoffes enzymatisch abgefangen werden. Bei Betrachtung der möglichen Verdünnung des Sauerstoffes aus der wässrigen Phase in die Gasphase (wässrig:Gas Phasen-Verhältnis 1:10, dimensionslose Henry Volatilität Hcc = caq/cgas für O2 in Wasser: 0.0297 bei 25°C (Sander 2015)) wird deutlich, dass der molekulare Sauerstoff bereits an der Stelle seiner Erzeugung abgefangen und in das Substrat eingebunden wurde, bevor es in die Gasphase entwichen ist. Eine Optimierung des Oxygenierungs-Systems (zum Beispiel durch Erhöhung des Enzymgehaltes in der Zelle), ist möglich und würde den Anteil an extrahiertem Sauerstoff erhöhen.

### Beispiel 2:

### Technische Anwendung - Rohrbündelreaktor Konzept

Die technische Umsetzung der beschriebenen Erfindung erfolgt bevorzugt über ein Konzept, in dem ein beleuchteter Rohrbündelreaktor zur Anwendung kommt, welcher suspendierte oder immobilisierte phototrophe Zellen beinhaltet. Ein Scale-Up des Rohrbündelreaktors in einen technischen Maßstab erfolgt durch einfache Erhöhung der Anzahl an eingesetzter Mikrokapillare. Als spezielle Form immobilisierter Zellen wird ein Biofilm-basierter Aufbau gewählt, welcher sich bereits für die Cyanobakterien-Spezies *Synechocystis sp.* PCC6803 als realisierbar erwies (David, C., K. Buhler and A. Schmid (2015). "Stabilization of single species Synechocystis biofilms by cultivation under segmented flow." J Ind Microbiol Biotechnol 42(7): 1083-1089). Diese technische Umsetzung bietet ein kontinuierliches Produktionssystem, welches die photosynthetische Wasserspaltung, die WasserstoffProduktion, die Sauerstoff Extraktion, sowie die Substrat Oxidation beinhaltet. Die nachgelagerte Aufbereitung wird dann durch das Abfangen des molekularen Wasserstoffes über die Gasphase und, bei Einsatz von Abwasser als Substrat, durch Rückführung des behandelten Abwassers zum weiterführenden Aufbereitungsprozess ermöglicht. Wird hingegen ein kombinierter biokatalytischer Produktionsprozess beabsichtigt, ist eine Abtrennung des Produktes mit erhöhter Wertschöpfung zum Beispiel durch den Einsatz einer organischen Trägerphase durchzuführen. Die folgenden Beispiele enthalten Annahmen und Werte welche entsprechend der spezifischen Anwendung und der gewählten Rahmenbedingungen angepasst werden und zeigen das Potential der Erfindung.

### Spezifische Aktivität bezüglich der Wasserspaltung durch das PSII

Die verwendeten Mikroorganismen weisen einen Gehalt von ca. 1% des Photosystems II auf, wobei das Photosystem II eine molare Masse von 350 kDa besitzt (g_{CDW}⁻¹, Molekulargewicht PSII (Shen, J. R. (2015). "The structure ofphotosystem II and the mechanism of water oxidation in photosynthesis." Annu Rev Plant Biol 66: 23-48). Der höchste in vitro gemessene Wert wird von Dismukes et al. (Dismukes, G. C., R. Brimblecombe, G. A. Felton, R. S. Pryadun, J. E. Sheats, L. Spiccia and G. F. Swiegers (2009). "Development of bioinspired Mn4O4-cubane water oxidation catalysts: lessons from photosynthesis." Acc Chem Res 42(12): 1935-1943.) mit 1000 s⁻¹ angegeben. Dies führt zu einer spezifischen Aktivität des PSII von 1700 µmol_{H2Ospaltung} pro Minute je g_{CDW}⁻¹. Daraus ergibt sich bezüglich einer Wasserstoffproduktion, einem Sauerstoffverbrauch und einer Substartoxidierung durch den Organismus von 850µmol_{H2/O2/Substrat} pro Minute je g_{CDW}⁻¹.

### Produkt Ertrag durch einen Rohrbündelreaktor Prozess

Auf 20 000 Mikrokapillaren mit je 2 m Länge und 5 mm Durchmesser werden 10 g_{CDW} L⁻¹ Biomasse mit einer volumetrischen Produktivität von 0.51 mol_{H2/O2/Substrat} L⁻¹ h⁻¹ eingesetzt. Es ergibt sich ein Gesamtvolumen von 785 L (39.3 mL Kapillare⁻¹). Bei 2920 Sonnenstunden im Jahr (Licht Verfügbarkeit 8 h pro Tag, 365 Tage) produziert das System 1169 kmol Wasserstoff, sowie oxidiertes Substrat pro Jahr.

### Energieertrag

Es gilt die Voraussetzung, dass der Prozess nicht durch die Hydrogenase limitiert ist. Auf Basis eines Molekulargewichts von 2 g/mol weist Wasserstoff eine volumetrische Produktaktivität von 1.02 g_{H2} L⁻¹ h⁻¹. Daraus ergibt sich ein Produktertrag von 2338 kg Wasserstoff pro Jahr von denen 60 % also 1402 kg rückgewonnen werden. Bei einem zugrunde gelegten Energiegehalt von 33.3 kWh je Kilogramm Wasserstoff (Wikipedia) kann mit dem erfindungsgemäßen Prozess eine Energie von 46 686 kWh pro Jahr generiert werden.

## Patentansprüche

1. Verfahren zur bioreaktiven Extraktion erzeugten Sauerstoffs aus einem Reaktionsraum, umfassend die folgenden Schritte:
- Bestrahlen zumindest eines phototrophen Mikroorganismus unter anaeroben Bedingungen in einem Reaktionsraum, und
- in situ Binden von entstehendem Sauerstoff mittels des Mikroorganismus durch Oxidation eines Sauerstoff-verwertenden Substrats, wobei der zumindest eine phototrophe Mikroorganismus zumindest ein Sauerstoff-umsetzendes Enzym aufweist oder produziert.

2. Verfahren nach Anspruch 1, wobei das zumindest eine Sauerstoff-umsetzende Enzym ausgewählt ist aus der Gruppe Hydrogenase, Nitrogenase und/oder Oxidoreduktase.

3. Verfahren nach einem der Ansprüche 1 bis 2, wobei bei der Bestrahlung des zumindest einen phototrophen Mikroorganismus neben Sauerstoff auch Wasserstoff oder Methanol frei wird.

4. Verfahren nach einem der Ansprüche 2 bis 3, **dadurch gekennzeichnet, dass** die Oxidoreduktasen ausgewählt sind aus der Gruppe der Oxidasen oder Oxygenasen, vorzugsweise Monooxygenasen, Dioxygenasen oder coenzym-unabhängige Oxygenasen.

5. Verfahren nach einem der vorhergehenden Ansprüche, wobei als phototropher Mikroorganismus Algen oder Cyanobakterien eingesetzt werden, vorzugsweise Cyanobakterien.

6. Verfahren nach einem der vorhergehenden Ansprüche, **dadurch gekennzeichnet, dass** als Mikroorganismus ein, insbesondere genetisch modifizierter, Cyanobakterienstamm eingesetzt wird, der das Alkan-Monooxygenase-Enzymsystem AlkBGT aufweist.

7. Verfahren nach einem der vorhergehenden Ansprüche, **dadurch gekennzeichnet, dass** der genetisch modifizierte Cyanobakterienstamm Synechocystis sp. PCC6803 eingesetzt wird, der das Alkan-Monooxygenase-Enzymsystem AlkBGT aufweist oder produziert.

8. Verfahren nach Anspruch 7, **dadurch gekennzeichnet, dass** als Substrat Methylnonanoat verwendet wird.

## Claims

1. A process for the bioreactive extraction of produced oxygen from a reaction chamber, comprising the following steps:
- irradiating at least one phototrophic microorganism under anaerobic conditions in a reaction chamber and
- in situ binding of developing oxygen by means of the microorganism through oxidation of an oxygen-utilizing substrate, wherein the at least one phototrophic microorganism has or produces at least one oxygen-converting enzyme.

2. The process according to Claim 1, wherein the at least one oxygen-converting enzyme is selected from the group of hydrogenase, nitrogenase and/or oxidoreductase.

3. The process according to either one of Claims 1 to 2, wherein, during the irradiation of the at least one phototrophic microorganism, hydrogen or methanol is also released in addition to oxygen.

4. The process according to either one of Claims 2 to 3, **characterized in that** the oxidoreductases are selected from the group of oxidases or oxygenases, preferably monooxygenases, dioxygenases or coenzyme-independent oxygenases.

5. The process according to any one of the preceding claims, wherein algae or cyanobacteria, preferably cyanobacteria, are used as the phototrophic microorganism.

6. The process according to any one of the preceding claims, **characterized in that** a, in particular genetically modified, strain of cyanobacteria which has the alkane monooxygenase enzyme system AlkBGT is used as the microorganism.

7. The process according to any one of the preceding claims, **characterized in that** the genetically modified strain of cyanobacteria Synechocystis sp. PCC6803 which has or produces the alkane monooxygenase enzyme system AlkBGT is used.

8. The process according to Claim 7, **characterized in that** methyl nonanoate is used as the substrate.

## Revendications

1. Procédé d'extraction bioréactive d'oxygène généré à partir d'une chambre de réaction, comprenant les étapes suivantes :
- l'exposition à un rayonnement d'au moins un microorganisme phototrophe dans des conditions anaérobies dans une chambre de réaction, et
- la fixation in situ de l'oxygène produit au moyen du microorganisme par oxydation d'un substrat utilisant l'oxygène, l'au moins un microorganisme phototrophe comprenant ou produisant au moins une enzyme de conversion de l'oxygène.

2. Procédé selon la revendication 1, dans lequel l'au moins une enzyme de conversion de l'oxygène est choisie dans le groupe des hydrogénases, des nitrogénases et/ou des oxydoréductases.

3. Procédé selon l'une quelconque des revendications 1 à 2, dans lequel, lors de l'exposition à un rayonnement de l'au moins un microorganisme phototrophe, de l'hydrogène ou du méthanol est libéré en plus de l'oxygène.

4. Procédé selon l'une quelconque des revendications 2 à 3, **caractérisé en ce que** les oxydoréductases sont choisies dans le groupe des oxydases ou des oxygénases, de préférence des monooxygénases, des dioxygénases ou des oxygénases indépendantes des coenzymes.

5. Procédé selon l'une quelconque des revendications précédentes, dans lequel des algues ou des cyanobactéries, de préférence des cyanobactéries, sont utilisées en tant que microorganisme phototrophe.

6. Procédé selon l'une quelconque des revendications précédentes, **caractérisé en ce qu'**une souche de cyanobactéries, notamment génétiquement modifiée, qui présente le système enzymatique alcane-monooxygénase AlkBGT est utilisée en tant que microorganisme.

7. Procédé selon l'une quelconque des revendications précédentes, **caractérisé en ce que** la souche de cyanobactéries génétiquement modifiée Synechocystis sp. PCC6803, qui comprend ou produit le système enzymatique alcane-monooxygénase AlkBGT, est utilisée.

8. Procédé selon la revendication 7, **caractérisé en ce que** du nonanoate de méthyle est utilisé en tant que substrat.
